# EUROPEAN PATENT SPECIFICATION

(11) **EP 0 856 158 B1**
(45) Date of publication and mention of the grant of the patent: **09.02.2000**
(21) Application number: 96931219.8
(22) Date of filing: 26.09.1996
(51) Int. Cl.: G01N 33/68, G01N 33/564, G01N 33/569

(54) **ASSAY FOR THE DIAGNOSIS OF DEMENTIA**
TEST ZUR DIAGNOSE VON DEMENZ
DOSAGE PERMETTANT DE DIAGNOSTIQUER LA DEMENCE

(30) Priority: 29.09.1995 IL 11546595
(43) Date of publication of application: 05.08.1998
(73) Proprietor: YEDA RESEARCH AND DEVELOPMENT Co. LTD., Rehovot 76100 (IL)
(72) Inventor: SHINITZKY, Meir, 46544 Kfar-Shmaryahu (IL); DECKMANN, Michael, F-68500 Guebwiller (FR)
(74) Representative: VOSSIUS & PARTNER
(86) International application number: IL9600118
(87) International publication number: WO9713152

(56) References cited:
- WO-A-91/15773
- WO-A-93/10459
- WO-A-95/12685
- WO-A-95/23970
- AN. N.Y. ACAD. SCI., vol. 621, 1991, pages 205-217, XP002024437 SHINITZKY, M. ET AL.: "Platelet autoantibodies in dementia and schizophrenia - possible implication for mental disorders" cited in the application

## Description

### FIELD OF THE INVENTION

The present invention relates to an assay for the diagnosis of multi-infarct dementia (MID) and dementia of the Alzheimer type (DAT).

### BACKGROUND OF THE INVENTION

Dementia of the Alzheimer type (DAT) is the most common cause of dementia in the elderly (Evans, D.A., *et al., J. Am. Med. Assoc.,* **262**:2551 (1989)).

The pathogenesis of DAT involves accumulation of amyloid and paired helical filaments (PHF) in cerebral tissues as well as synaptic loss, both leading to neuronal death which eventually results in mental deterioration (Massimo Tabaton, M., *Alzheimer's Disease Update,* **10**:249 (1994)).

The clinical diagnosis of DAT is confounded by other dementing diseases, particularly those common in the elderly, having similar clino-pathological features. One such dementia which cannot be effectively separated by diagnosis from DAT is multi-infarct dementia (MID) in which cerebral tissue damage seems to be caused mainly by breakage of small cerebral vessels leading to clino-pathologic results which are similar to those detected in DAT.

At least some forms of DAT have a genetic etiology and, recently, three different genes have been reported in which mutations related to this disease were identified (Levy-Lahad, E., *Science,* **269:**973 (1995)). Identification of such genes will no doubt contribute to the understanding of the pathogenesis of the disease, but as of today, these findings are not yet utilized for diagnosis of DAT.

The existence of elevated levels of auto antibodies on blood platelets of various demented patients has been previously reported (Shinitzky, *M. et al., An. N. Y. Acad. Sci.,* **621**:205-217 (1991)). However, these findings, as well, could not be used for diagnosis of MID or DAT.

The diagnosis of DAT today is carried out by an exclusion process, i.e. by excluding possible other dementias and remaining with a high probability that the dementia in question is indeed DAT. Confirmation of such diagnosis is possible only by performing postmortem histopathological examination of brain tissues. Therefore, in actuality, no diagnostic assay for diagnosing MID and DAT is yet available.

Neuronal cell death, which occurs in MID and DAT patients, is a non reversible phenomena. Therefore, it is clearly highly desirable to provide an assay for early diagnosis of MID and DAT on the basis of which it would become possible to detect individuals having a high risk of developing these diseases. Such individuals could then be treated with various medications intended to delay or, if possible, to prevent, the clinical processes causing the onset of these diseases.

### SUMMARY OF THE INVENTION

The present invention is based on the surprising finding that patients suffering from multi-infarct dementia (MID) and dementia of the Alzheimer type (DAT), display a high level of a 75 kD platelet protein and/or a high level of platelet-associated antibodies against said protein as compared to normal subjects.

This surprising finding paved the way to the development of an assay for the detection of MID and DAT by detecting the levels of the 75 kD platelet protein, the level of platelet-associated antibodies against this protein or the levels of both, and comparing said level or levels to those of a normal control, a higher level indicating a likelihood that the tested individual has MID and/or DAT.

According to one aspect of the invention termed *"the antibody aspect"* the present invention provides an assay for the diagnosis of multi-infarct dementia and dementia of the Alzheimer type in an individual comprising the steps of:
i. obtaining a sample from said individual being a blood sample, a platelet-containing fragment thereof, or a fragment containing platelet associated antibodies (PAA) detached from the platelets;
ii. determining the level of a platelet associated antibody which is capable of binding to a platelet-protein in said sample, said platelet-protein having a molecular weight of about 75 kD as determined by gel electrophoresis and having a pI in a pH range of about 7-9, a level higher than that of a control sample indicating that said individual has a high likelihood of having multi-infarct dementia or dementia of the Alzheimer type.

According to another aspect of the present invention termed *"the protein aspect"* the present invention provides an assay for the diagnosis of multi-infarct dementia and dementia of the Alzheimer type an individual comprising the steps of:
i. obtaining a sample from said individual being a blood sample or a platelet-containing fragment thereof;
ii. determining the level of a 75 kD platelet-protein in said sample, said platelet-protein having a molecular weight of about 75 kD as determined by gel electrophoresis and having a pI in a pH range of about 7-9, a level higher than that of a control sample indicating that said individual has a high likelihood of having multi-infarct dementia and dementia of the Alzheimer type.

According to yet another aspect of the invention termed *'the antibody and protein aspect"* the invention provides an assay for the diagnosis of multi-infarct dementia and dementia of the Alzheimer type in an individual comprising the steps of:
i. obtaining a sample from said individual being a blood sample or a platelet-containing fragment thereof;
ii. determining the level of a platelet-associated antibody which is capable of binding to a platelet protein having a molecular weight of about 75 kD as determined by gel electrophoresis and having a pI in a pH range of about 7-9, and the level of said platelet-protein in said sample, levels being higher than the corresponding two levels of a control sample indicating that said individual has a high likelihood of having multi-infarct dementia and dementia of the Alzheimer type.

The assay of the invention is suitable for detecting both multi-infarct dementia, and dementia of the Alzheimer type since, as explained above, the clinical symptoms of the two types of dementia are usually indistinguishable. As acceptable today, all elderly patients suffering from DAT are believed to be also effected by MID.

The sample of the tested individual may be a blood sample such as serum; a platelet-containing fraction thereof such as platelet rich plasma (PRP) obtained by treatment of the blood with an anti-coagulant e.g. heparin or sodium citrate and centrifugation after which platelets may be obtained from the pelleted fraction; or, in the antibody aspect of the invention, a blood sample fraction containing PAA detached from the obtained platelets.

According to the antibody aspect of the invention where the level of the PAA against the 75 kD platelet protein is to be determined, the antibodies are obtained by centrifugation of PRP, incubation of the pelleted platelets with glycin HCl buffer pH 2.5-3.0 at room temperature and centrifugation, thus obtaining the PAA-containing supernatant. The level of the PAA against the specific 75 kD platelet protein level is then determined as will be explained hereinbelow.

According to the protein aspect of the invention, the platelet proteins are obtained by separation of the platelets from the sample, for example, by obtaining PRP as explained above, and then solubilizing the platelets and separating their proteins by methods well known in the art such as, for example, by polyacrilamide gel electrophoresis (PAGE) by affinity chromatography, high-performance liquid chromatography (HPLC) etc. The level of the 75 kD protein obtained is determined as will be explained hereinbelow.

According to the antibody and protein aspect of the invention, both the PAA and platelet proteins are separately obtained from the sample and the level of the PAA against the 75 kD platelet-protein as well as the level of the protein itself are separately determined.

An example of a method for determination of the level of the PAA against the 75 kD platelet-protein is by immobilization of the 75 kD protein, or an antibody binding fragment of said protein, by their binding to a solid support such as nitrocellulose, or various kinds of inert beads and then contacting the immobilized proteins with the PAA containing sample under conditions allowing antibody-antigen binding (Shinitzki *et al., ibid.).* Unbound antibodies are removed and the level of bound antibodies is detected, for example, by the use of anti-human immunoglobulin antibodies or an antigen binding fragment thereof linked to a marker. Such a marker may be for example, a radioactive group; a fluorescent group; an enzyme that can catalyze a reaction yielding a detectable product such as, for example, horseradish peroxidase (HRP) and alkaline phosphatase; a biotin group that can be detected by streptavidin, etc.

Another example of a method for determining the level of PAA against the 75 kD platelet-protein is by use of anti-idiotype antibodies raised by immunizing an animal with a purified preparation of PAA. According to this method, the PAA antibodies of the sample are immobilized onto a solid support, for example, by their binding to anti-human immunoglobulin antibodies. The obtained anti-idiotype antibodies are then contacted with the immobilized PAA under conditions allowing antibody-binding and unbound antibodies are removed. The anti-idiotype antibodies may be directly labelled with detectable markers such as radioactive, fluorescent or enzymatic groups. Alternatively, the level of the bound anti-idiotype antibodies may be determined by contacting the first unlabelled bound anti-idiotype antibodies with a secondary labelled species specific anti-immunoglobulin antibody (directed against the species of the anti-idiotype producing animal).

The separation and immobilization of the platelet-proteins can be carried out by electrophoretic separation of the proteins on a polyacryamid gel, for example, a 7.5-15% acrylamid gradient gel, and then the transfer of the separated proteins onto a nitrocellulose sheet. The detection of the 75 kD platelet-protein can then be achieved by contacting the protein containing sheet with anti-75kD platelet-protein PAA as described above, (i.e. by the technique known as *"western blotting").*

The 75 kD platelet protein may also be detected, for example, by use of affinity binding assays wherein the binding molecules used are various molecules such as receptors, enzymes, etc., capable of specifically binding and recognizing the 75 kD platelet protein.

Once the level of the antibody, the protein or both the antibody and the protein have been determined, their level is compared to the corresponding level or levels of the antibody and/or protein in a control sample.

A control sample may be blood samples of the type defined above pooled from several individuals which do not feature symptoms of dementia. It is essential not to use as a control a sample from a single person even if he is considered healthy, since about 25% of the population will eventually suffer from dementia and it is believed that the onset of the disease occurs decades before the clinical symptoms of dementia appear. It may well be, therefore, that during this symptom free period the level of the 75 kD platelet-protein or the level of PAA against it already begin to change.

Alternatively, it is possible to use as a control sample a pool of samples from individuals in which no anti 75 kD platelet-protein PAA were detected.

A high determined-level of the anti 75 kD platelet-protein PAA or 75 kD platelet-protein itself, or both, in a tested individual as compared to the corresponding level of the antibody and/or protein in the control sample, indicates a likelihood of the individual having MID or DAT.

Experimental results obtained in accordance with the present invention showed that, while essentially all dementia patients show raised level of the anti 75 kD platelet-protein antibody, the protein itself or both, so did a small percentage of healthy individuals. While this may be a false positive result, it may also be explained by the fact that the level of the 75 kD platelet-protein or the PAA antibody against it is indicative of future development of dementia in an individual. Thus, a positive result indicates a high likelihood of having MID or DAT, either given the person is currently suffering from these types of dementia or given he will be effected by these types of dementia later in life.

The present invention further comprises a kit for use in the assays of the invention comprising substances and reagents required for determining the level of the 75 kD platelet-protein, the PAA against the 75 kD platelet-protein or both, as the case may be.

The invention will now be illustrated with reference to some nonlimiting examples.

### BRIEF DESCRIPTION OF THE FIGURES

**Fig. 1** shows a photograph of a Western blot in which PAA from demented patients (Fig. 1A) from elderly normal subjects (Fig. 1B) and from normal young to middle aged subjects (Fig. 1C) were incubated with normal human platelet-proteins.

**Fig. 2A** shows a photograph of a Western blot prepared from a pool of platelets derived from healthy subjects probed with PAA from demented patients.

**Fig. 2B** shows a photograph of a Western blot of a pool of platelets derived from demented patients probed with PAA from demented patients.

**Fig. 2C** shows a photograph of a Western blot of a pool of platelets derived from normal subjects probed with PAA from demented patients.

**Fig. 2D** shows a photograph of a Western blot prepared from a pool of platelets derived from demented patients and probed with PAA from normal subjects.

**Fig. 3** shows a photograph of a blot showing platelet-proteins of a pool of platelets derived from 60 blood donors after preparative isoelectric focusing of the proteins (see above) and incubation with anti-75 kD protein PAA.

### DETAILED DESCRIPTION OF THE INVENTION

### General Procedures

### i. Patients and control subjects

Young (< 65 years) and healthy volunteers, old (> 65)) non-demented volunteers and old (> 65 years) demented patients participated in this study. The patients and the old non-demented volunteers were members of the Geriatric Hospital, Pardes Hanna, Israel, whereas the young volunteers were staff members of the Geriatric Hospital, Pardes Hanna, Israel and The Weizmann Institute of Science, Rehovot, Israel. For the demented patients participating in this study, all other organic reactions except for multi-infarct dementia (MID) and dementia of the Alzheimer type (DAT) were excluded.

### ii. Preparation of Platelet Rich Plasma (PRP)

Venous blood was drawn in the morning using heparin as anticoagulant. PRP was obtained from full blood by centrifugation (100 x g, for 15 mins.) at room temperature (20-24°C). The number of platelets in the PRP were scored microscopically.

### iii. Isolation of Platelet Associated Antibodies (PAA) from platelets

4 to 15 ml of PRP were centrifuged (2000 x g for 20 mins) at room temperature. The supernatant was discarded and the pellet was resuspended in 5 ml PBS/10 mM EDTA and centrifuged again. This step was repeated three times. For the removal of PAA from the pelleted platelets, the platelets were resuspended in 1 ml 150 mM glycine/HCl buffer, pH 2.7, for 10 mins. at room temperature. The platelets were then pelleted by centrifugation (2000 x g for 15 mins.) and the PAA containing supernatant was collected and immediately neutralized to pH 7 with the appropriate amount of a saturated solution of Na₂PHO₄.

### iv. Polyacrylamide gel electrophoresis (PAGE)

Platelets were derived from PRP by three washings with PBS/10 mM EDTA and separated by 10% PAGE according to their molecular weights as described in Laemmli, U.K., *Nature.,* 227, 680 (1970) and in Shinitzky *et al., ibid.*

### v. Western blot

For the electrophoretic transfer of proteins, from the gel (see (iv) above) onto a nitrocellulose sheet, the 10% PAGE was placed between sheets of nitrocellulose or PVDF membranes, Whatmann filter paper No. 4 and pads as holders. The transfer was performed in a Western blot cell (BioRad), filled with transfer buffer. Two transfer buffers were used: for transfer in two hours by 750 mA at 10°C, the buffer consisted of 0.025M Tris, 0.192M glycine, 0.1% SDS and 20% methanol. For transfer overnight (about 12 hours) by 100 mA at 10°C, the buffer consisted of 10 mM Caps, pH 11 and 10% methanol. The efficiency of the protein transfer was monitored by a nitrocellulose sheet stained with the reversibly-binding dye Poinceau S. After removal of the Poinceau S stain with double distilled water, the nitrocellulose sheet was air-dried, cut into 0.3 cm wide strips and stored dry at room temperature until use.

### vi. Western blot analysis

The proteins which were transferred into the nitrocellulase sheet ((v) above) were probed as follows: All incubation steps were carried out at room temperature and under constant shaking (Bellco Rocking Table. speed setting 4) in BioRad Incubation Trays. The strips were first incubated for 5 mins. in 1 ml incubation buffer (6() mM citric acid. 90 mM Na₂PHO₄. 0.3% Tween 20, 200 mM NaCl, pH 7.7) in order to wet and block the strips. Then, the sample (e.g. 0.2 ml PAA, 0.1 ml plasma) was added and incubated for 14 hours. After the incubation, the strips were washed 3 times with 1 ml incubation buffer and one time with 1 ml peroxidase buffer (200 mM Tris, 0.3% Triton X-100, 10 mM phenol, 2 mM CaCl₂, pH 8.0 for 15 mins. The strips were then incubated with goat anti-human F_{C}-IgG covalently linked to horse radish peroxidase (Bio Makor, Israel) in a dilution of 1:250 in 1 ml per strip for 2 hours. The strips were then washed 3 times with 1 ml peroxidase buffer and one time with 1 ml double distilled water for 10 mins. Bound peroxidase was visualized by incubating the strips with 1 ml color regent (12 mg 4-chloronaphthol, 4 ml methanol, 20 ml PBS, 0.005 ml 30% H₂O₂) for 30 to 60 mins. The color reaction was stopped by washing the strips with double distilled water.

### vii. Preparative isoelectric focusing

Platelet Rich Plasma from 60 blood bank donors (blood group 0) were pooled and extensively washed with PBS/10 mM EDTA until the supernatant was free of plasma proteins. The platelets were then solubilized with 1% NP40 in DDW. The unsolubilized material was pelleted by centrifugation at 10,000 g for 15 mins. at 4°C. The unsolubilized was then repeatedly treated with 1% Triton X-100 in DWW until the supernatant became poor in protein. All supernatants were pooled and isoelectric focused in a ROTOFOR apparatus (BioRad) according to the instructions of the manufacturer. A pH gradient of 1.5 to 12.5 was generated using ampholyte 3/10 from BioRad. After focusing, the fractions were analyzed for pH, protein pattern and relevant proteins.

### Example 1

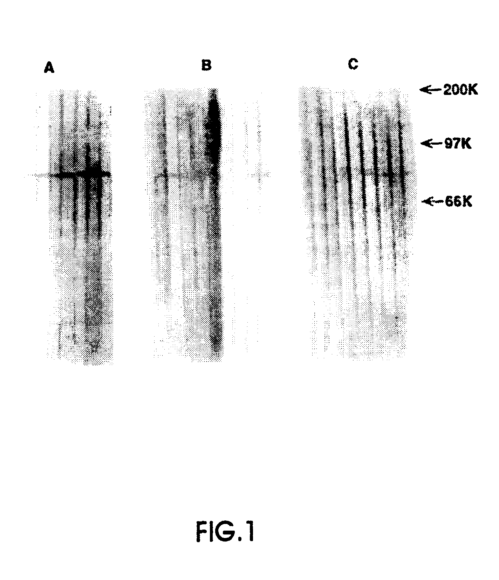

PAA from 9 demented patients, 10 old but normal subjects and 9 young to middle aged normal subjects were incubated with normal human platelet proteins, separated by gel electrophoresis and blotted onto nitrocellulose as described above. Visual detection of bound PAA was carried out as described above. The results are shown in Fig. 1, wherein molecular weight markers from BioRad were used and the arrows indicate the positions of the 66 kD, 97 kD and the 200 kD markers.

With PAA from the demented patients, a protein with an apparent molecular weight of about 75 kD was detected in all cases (Fig. 1A). An additional protein with an apparent molecular weight of about 110 kD was detected in three cases (Fig. 1A). The 75 kD band could only faintly be detected in 3 out of 10 old non demented subjects (Fig. 1B) and 3 out of 9 young middle aged normal subjects (Fig. 1C). As explained above, this indication may not merely present a false positive but may indicate a tendency of acquiring MID or DAT later in life.

### Example 2

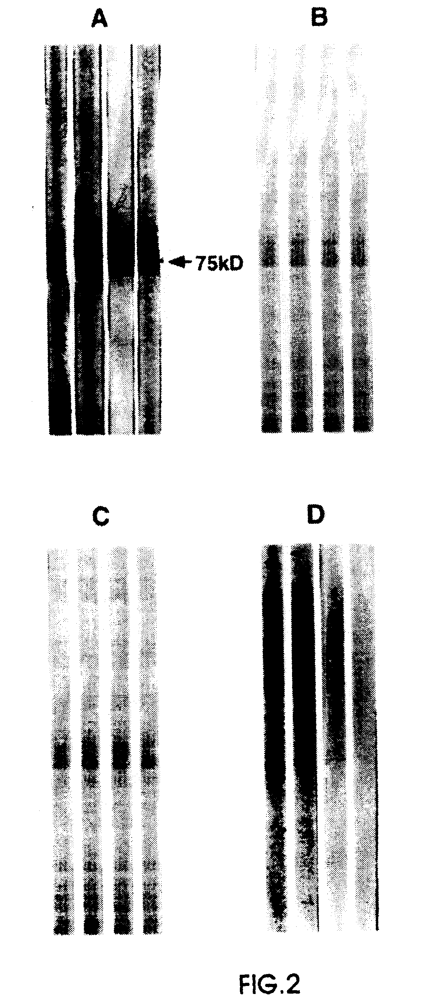

Platelets from healthy and normal subjects whose PRP did not contain detectable amounts of PAA against the 75 kD protein, determined as described in Example 1, were pooled. The platelets from demented patients were also separately pooled. The proteins of each of these two pools were separated by gel electrophoresis and blotted onto nitrocellulose as described above. The blot prepared from platelets of healthy subjects will be referred to hereinafter as the *"normal"* blot and the blot prepared from platelets of the demented patients will be referred to as the *"dementia"* blot. The *"dementia"* Western blot was then probed with PAA from demented patients, whereas the *"normal*" Western blot was probed with PAA from non demented subjects as described above. Under these conditions, the 75 kD band was not detected on the *"normal*" Western blot (Fig. 2A). However, this band became strongly visible, when PAA from demented patients was used to probe the *"dementia*" Western blot (Fig. 2B).

In order to determine whether the 75 kD protein is dementia specific, a cross-over experiment was conducted. The *"dementia"* Western blot was probed with PAA from normal subjects and the *"normal"* Western blot was probed with PAA from demented patients as described above. Surprisingly, the PAA from demented patients could not detect the 75 kD protein in Western blot of normal subjects (Fig. 2C)., the PAA from normal subjects did not at all or only faintly detect this protein in the Western blot of demented patients (Fig. 2D).

These results indicate that an autoantibody against the 75 kD protein exists in demented patients and that the 75 kD protein itself seems newly generated in demented patients and is absent in platelets from normal subjects.

### Example 3

Platelets from 60 blood donors (blood group 0) were isolated from platelet rich plasma as described above. The pooled platelets were solubilized and separated by isoelectric focusing as described above. The proteins of the different fractions ranging from pH 1.5 to 12.5, were separated by gel electrophoresis, blotted and screened for the 75 kD protein as described above.

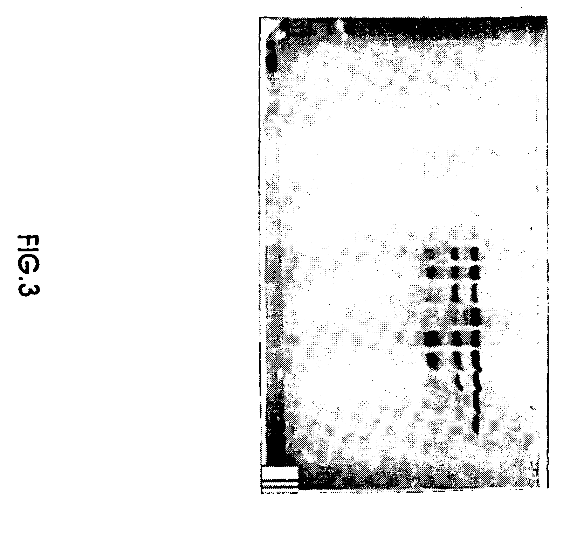

The result of this experiment are shown in Fig. 3. As can be seen, the 75 kD protein could be found in the pool from 60 blood donors as expected from a statistical point of view, since 25% of the elder people will suffer from dementia.

Fig. 3 also shows that the 75 kD protein has a pI between pH 7 to pH 9, which indicates that this protein exists in different isoforms and/or that there is a family of several proteins with the same molecular weight but different pI involved.

## Claims

1. An assay for the diagnosis of multi-infarct dementia and dementia of the Alzheimer type in an individual comprising the steps of:
i. obtaining a sample from said individual being a blood sample, a platelet-containing fragment thereof, or a fragment containing platelet associated antibodies (PAA) detached from the platelets;
ii. determining the level of a platelet associated antibody which is capable of binding to a platelet-protein in said sample, said platelet-protein having a molecular weight of about 75 kD as determined by gel electrophoresis and having a pI in a pH range of about 7-9, a level higher than that of a control sample indicating that said individual has a high likelihood of having multi-infarct dementia or dementia of the Alzheimer type.

2. An assay for the diagnosis of multi-infarct dementia and dementia of the Alzheimer type in an individual comprising the steps of:
i. obtaining a sample from said individual being a blood sample or a platelet-containing fragment thereof;
ii. determining the level of a 75 kD platelet-protein in said sample, having a molecular weight of about 75 kD as determined by gel electrophoresis and having a pI in a pH range of about 7-9, a level higher than that of a control sample indicating that said individual has a high likelihood of having multi-infarct dementia and dementia of the Alzheimer type.

3. An assay for the diagnosis of multi-infarct dementia and dementia of the Alzheimer type in an individual comprising the steps of:
i. obtaining a sample from said individual being a blood sample or a platelet-containing fragment thereof;
ii. determining the level of a platelet-associated antibody which is capable of binding to a platelet protein in said sample, said platelet-protein having a molecular weight of about 75 kD as determined by gel electrophoresis and having a pI in a pH range of about 7-9, and the level of said platelet-protein in said sample, levels being higher than the corresponding two levels of a control sample indicating that said individual has a high likelihood of having multi-infarct dementia and dementia of the Alzheimer type.

4. The assay of Claims 1 or 3, wherein the determination of the level of the antibody comprises:
(a) immobilizing the 75 kD platelet-protein or an antibody-binding fragment thereof, onto a solid support;
(b) contacting the sample with the immobilized protein under conditions allowing antibody-antigen binding;
(c) removing unbound antibodies from the solid support; and
(d) determining the level of antibody bound onto the solid support.

5. The assay of Claim 4, wherein the determination of step (d) comprises adding to the bound antibodies anti-human immunoglobulin antibodies conjugated to a detectable marker.

6. The assay of Claims 2 or 3, wherein determination of the 75 kD platelet protein level comprises:
(a) immobilizing the sample's proteins onto a solid support;
(b) contacting the immobilized proteins with platelet-associated antibodies capable of specifically binding to the 75 kD protein or with antigen-binding fragments thereof of said antibodies;
(c) removing unbound antibodies from the solid support;
(d) determining the level of bound antibodies.

7. The assay of Claim 6, wherein the determination of step (d) comprises adding to the bound antibodies anti-human immunoglobulin antibodies conjugated to a detectable marker.

8. The assay of Claim 6, wherein the immobilization of step (a) comprises electrophoretically separating the sample's proteins on a polyacrylamide gel and then immobilizing said separated protein on to a nitrocellulose support.

9. The assay of Claim 8, wherein the gel is a 7.5-15% acrylamide gradient gel.

10. A kit comprising
(a) the platelet-protein having a molecular weight of about 75 kD as determined by gel electrophoresis and a pl in a pH range of about 7-9; and or
(b) the platelet-associated antibody (PAA) against the protein of (a).

## Patentansprüche

1. Test zur Diagnose der Multiinfarktdemenz und der Demenz des Alzheimertyps in einem Individuum, umfassend folgende Schritte:
i. Erhalten einer Probe von dem Individuum, die eine Blutprobe, ein Blutplättchen enthaltendes Fragment davon oder ein Fragment ist, das von Blutplättchen abgelöste Blutplättchen-assoziierte Antikörper (PAA) enthält;
ii. Bestimmen der Menge eines Blutplättchen-assoziierten Antikörpers, die fähig ist, an Blutplättchenprotein in der Probe zu binden, wobei das Blutplättchenprotein ein Molekulargewicht von etwa 75 kD, wie durch Gelelektrophorese bestimmt, und einen pl-Wert in einem pH-Bereich von etwa 7-9 hat, wobei eine Menge, die höher ist als jene einer Kontrollprobe, anzeigt, daß das Individuum mit hoher Wahrscheinlichkeit eine Multiinfarktdemenz oder eine Demenz des Alzheimertyps hat.

2. Test zur Diagnose der Multiinfarktdemenz und der Demenz des Alzheimertyps in einem Individuum, umfassend die folgenden Schritte:
i. Erhalten einer Probe von dem Individuum, die eine Blutprobe oder ein Blutplättchen enthaltendes Fragment davon ist;
ii. Bestimmen der Menge eines 75 kD-Blutplättchenproteins in der Probe, das ein Molekulargewicht von etwa 75 kD, wie durch Gelelektrophorese bestimmt, und einen pl-Wert in einem pH-Bereich von etwa 7-9 hat, wobei eine Menge, die höher ist als jene einer Kontrollprobe, anzeigt, daß das Individuum mit hoher Wahrscheinlichkeit eine Multiinfarktdemenz oder eine Demenz des Alzheimertyps hat.

3. Test zur Diagnose der Multiinfarktdemenz und der Demenz des Alzheimertyps in einem Individuum, umfassend die folgenden Schritte:
i. Erhalten einer Probe von dem Individuum, die eine Blutprobe oder ein Blutplättchen enthaltendes Fragment davon ist;
ii. Bestimmen der Menge eines Blutplättchen-assoziierten Antikörpers, der fähig ist, ein Blutplättchenprotein der Probe zu binden, wobei das Blutplättchenprotein ein Molekulargewicht von etwa 75 kD, wie durch Gelelektrophorese bestimmt, und einen pl-Wert in einem pH-Bereich von etwa 7-9 hat, und Bestimmen der Menge des Blutplättchenproteins in der Probe, wobei Mengen, die höher sind als die entsprechenden zwei Mengen einer Kontrollprobe, anzeigen, daß das Individuum mit hoher Wahrscheinlichkeit eine Multiinfarktdemenz oder eine Demenz des Alzheimertyps hat.

4. Test nach Anspruch 1 oder 3, wobei das Bestimmen der Menge des Antikörpers umfaßt:
(a) Immobilisieren des 75 kD-Blutplättchenproteins oder eines Antikörperbindenden Fragmentes davon an einem festen Träger;
(b) Inkontaktbringen der Probe mit dem immobilisierten Protein unter Bedingungen, die Antikörper-Antigen-Bindungen erlauben;
(c) Entfernen von ungebundenen Antikörpern vom festen Träger;
(d) Bestimmen der Menge des Antikörpers, die an den festen Träger gebunden ist.

5. Test nach Anspruch 4, wobei das Bestimmen von Schritt (d) das Hinzufügen von anti-menschliches Immunoglobulin-Antikörpern, konjugiert mit einem nachweisbaren Marker, zu den gebundenen Antikörpern umfaßt.

6. Test nach Anspruch 2 oder 3, wobei die Bestimmung der Menge des 75 kD-Blutplättchenproteins umfaßt:
(a) Immobilisieren der Proteine der Probe an einem festen Träger;
(b) Inkontaktbringen der immobilisierten Proteine mit Blutplättchen-assoziierten Antikörpern, die fähig sind, spezifisch das 75 kD-Protein oder mit Antigen-bindenden Fragmenten der Antikörper zu binden;
(c) Entfernen von ungebundenen Antikörpern vom festen Träger;
(d) Bestimmen der Menge der gebundenen Antikörper.

7. Test nach Anspruch 6, wobei das Bestimmen von Schritt (d) das Hinzufügen von anti-menschliches Immunoglobulin-Antikörpern, konjugiert mit einem nachweisbaren Marker, zu den gebundenen Antikörpern umfaßt.

8. Test nach Anspruch 6, wobei das Immobilisieren von Schritt (a) die elektrophoretische Trennung der Proteine der Probe in einem Polyacrylamidgel und anschließend das Immobilisieren des getrennten Proteins an einem Nitrocelluloseträger umfaßt.

9. Test nach Anspruch 8, wobei das Gel ein 7,5-15 %iges Acrylamid-Gradientengel ist.

10. Kit, umfassend:
(a) das Blutplättchenprotein, das ein Molekulargewicht von etwa 75 kD, wie durch Gelelektrophorese bestimmt, und einen pl-Wert in einem pH-Bereich von etwa 7-9 hat; und/oder
(b) den Blutplättchen-assoziierten Antikörper (PAA) gegen das Protein von (a).

## Revendications

1. Dosage pour le diagnostic de la démence multi-infarctus et de la démence de type Alzheimer chez un individu, comprenant les étapes consistant :
i. à prélever un échantillon dudit individu, lequel échantillon est un échantillon sanguin, un de ses fragments contenant des plaquettes, ou un fragment contenant des anticorps associés aux plaquettes (PAA), détachés des plaquettes ;
ii. à déterminer la quantité d'un anticorps associé aux plaquettes, qui est capable de se fixer à une protéine plaquettaire dans ledit échantillon, ladite protéine plaquettaire ayant une masse moléculaire d'environ 75 kD telle que déterminée par électrophorèse sur gel et ayant un pI sur une gamme de pH d'environ 7 à 9, une quantité supérieure à celle d'un échantillon témoin indiquant que ledit individu a une grande probabilité de présenter une démence multi-infarctus ou une démence de type Alzheimer.

2. Dosage pour le diagnostic pour la démence multi-infarctus et de la démence de type Alzheimer chez un individu, comprenant les étapes consistant :
i. à prélever un échantillon dudit individu, lequel échantillon est un échantillon sanguin ou l'un de ses fragments contenant des plaquettes ;
ii. à déterminer la quantité d'une protéine plaquettaire de 75 kD dans ledit échantillon, ayant une masse moléculaire d'environ 75 kD telle que déterminée par électrophorèse sur gel et ayant un pI sur une gamme de pH d'environ 7 à 9, une quantité supérieure à celle d'un échantillon témoin indiquant que ledit individu a une grande probabilité de présenter une démence multi-infarctus et une démence de type Alzheimer.

3. Dosage pour le diagnostic d'une démence multi-infarctus et de la démence de type Alzheimer chez un individu, comprenant les étapes consistant :
i. à prélever un échantillon dudit individu, lequel échantillon est un échantillon sanguin ou l'un de ses fragments contenant des plaquettes ;
ii. à déterminer la quantité d'un anticorps associé aux plaquettes, qui est capable de se fixer à une protéine plaquettaire dans ledit échantillon, ladite protéine plaquettaire ayant une masse moléculaire d'environ 75 kD telle que déterminée par électrophorèse sur gel et ayant un pI dans une gamme de pH d'environ 7 à 9, et la quantité de ladite protéine plaquettaire dans ledit échantillon, des quantités supérieures aux deux quantités correspondantes d'un échantillon témoin indiquant que ledit individu a une grande probabilité de présenter une démence multi-infarctus et une démence de type Alzheimer.

4. Dosage selon les revendications 1 ou 3, dans lequel la détermination de la quantité de l'anticorps consiste :
(a) à immobiliser la protéine plaquettaire de 75 kD ou l'un de ses fragments de fixation à des anticorps, sur un support solide ;
(b) à mettre en contact l'échantillon avec la protéine immobilisée, dans les conditions permettant une fixation anticorps-antigène ;
(c) à enlever du support solide les anticorps non-fixés ; et
(d) à déterminer la quantité d'anticorps fixé au support solide.

5. Dosage selon la revendication 4, dans lequel la détermination de l'étape (d) consiste à ajouter aux anticorps fixés des anticorps immunoglobulines anti-humains conjugués à un marqueur détectable.

6. Dosage selon les revendications 2 ou 3, dans lequel la détermination de la quantité de la protéine plaquettaire de 75 kD consiste :
(a) à immobiliser les protéines de l'échantillon sur un support solide ;
(b) à mettre en contact les protéines immobilisées avec des anticorps associés à des plaquettes, capables de se fixer d'une manière spécifique à la protéine de 75 kD ou avec ses fragments de fixation à un antigène, desdits anticorps ;
(c) à enlever dudit support solide les anticorps non-fixés ;
(d) à déterminer la quantité des anticorps fixés.

7. Dosage selon la revendication 6, dans lequel la détermination de l'étape (d) consiste à ajouter aux anticorps fixés des anticorps immunoglobuline anti-humains conjugués à un marqueur détectable.

8. Dosage selon la revendication 6, dans lequel l'immobilisation de l'étape (a) comprend la séparation électrophorétique des protéines de l'échantillon sur un gel de polyacrylamide, puis l'immobilisation de ladite protéine ainsi séparée sur un support de nitrocellulose.

9. Dosage selon la revendication 8, dans lequel le gel est un gel à gradient d'acrylamide de 7,5 à 15 %.

10. Trousse comprenant :
(a) une protéine plaquettaire ayant une masse moléculaire d'environ 75 kD telle que déterminée par électrophorèse sur gel et un pI dans une gamme de pH d'environ 7 à 9 ; et/ou
(b) l'anticorps associé aux plaquettes (PAA) contre la protéine (a).
